# EUROPEAN PATENT SPECIFICATION

(11) **EP 2 160 208 B1**
(45) Date of publication and mention of the grant of the patent: **06.03.2013**
(21) Application number: 08826554.1
(22) Date of filing: 16.05.2008
(51) Int. Cl.: A61L 29/12

(54) **MEDICAL BALLOONS AND THE METHODS OF MAKING THE SAME**
MEDIZINISCHE BALLONS UND VERFAHREN ZU IHRER HERSTELLUNG
BALLONNETS À USAGE MÉDICAL ET PROCÉDÉS POUR LES FABRIQUER

(30) Priority: 18.05.2007 US 804654
(43) Date of publication of application: 10.03.2010
(73) Proprietor: Boston Scientific Scimed, Inc., Maple Grove, MN 55311-1566 (US)
(72) Inventor: WEBER, Jan, 6228GJ Maastricht (NL); ATANSOSKA, Liliana, Edina, MN 55436 (US)
(74) Representative: Hauck Patent- und Rechtsanwälte
(86) International application number: PCT/US2008/063816
(87) International publication number: WO 2009/014793

(56) References cited:
- WO-A-01/34062
- WO-A-2006/107359
- WO-A-2008/024477
- WO-A2-2007/103356
- US-A1- 2005 181 014
- US-A1- 2007 207 182

## Description

### TECHNICAL FIELD

This disclosure relates to medical balloons, and to methods of making the same.

### BACKGROUND

The body includes various passageways such as arteries, other blood vessels, and other body lumens. These passageways sometimes become occluded, e.g., by a tumor or restricted by plaque. To widen an occluded body vessel, balloon catheters can be used, e.g., in angioplasty.

A balloon catheter can include an inflatable and deflatable balloon carried by a long and narrow catheter body. The balloon is initially folded around the catheter body to reduce the radial profile of the balloon catheter for easy insertion into the body.

During use, the folded balloon can be delivered to a target location in the vessel, e.g., a portion occluded by plaque, by threading the balloon catheter over a guide wire emplaced in the vessel. The balloon is then inflated, e.g., by introducing a fluid into the interior of the balloon. Inflating the balloon can radially expand the vessel so that the vessel can permit an increased rate of blood flow. After use, the balloon is deflated and withdrawn from the body.

In another technique, the balloon catheter can also be used to position a medical device, such as a stent or a stent-graft, to open and/or to reinforce a blocked passageway. For example, the stent can be delivered inside the body by a balloon catheter that supports the stent in a compacted or reduced-size form as the stent is transported to the target site. Upon reaching the site, the balloon can be inflated to deform and to fix the expanded stent at a predetermined position in contact with the lumen wall. The balloon can then be deflated and the catheter withdrawn. Stent delivery is further discussed in Heath, U.S. Patent No. 6,290,721.

One common balloon catheter design includes a coaxial arrangement of an inner tube surrounded by an outer tube. The inner tube typically includes a lumen that can be used for delivering the device over a guide wire. Inflation fluid passes between the inner and outer tubes. An example of this design is described in Arney et al., U.S. Patent No. 5,047,045.
In another common design, the catheter includes a body defining a guide wire lumen and an inflation lumen arranged side-by-side. Examples of this arrangement are described in Wang et al., U.S. Patent No. 5,195,969.

### SUMMARY

Generally, medical balloons are disclosed that include a balloon wall that includes a composite material that includes a polymeric material and a filler within the polymeric material. The polymeric material includes a reaction product of a first polymeric material that includes first polymer chains having a plurality of spaced first groups, and a second polymeric material that includes second polymer chains having a plurality of spaced apart second groups that react with the first groups. The first and second groups react by forming a covalent bond.

The disclosure features medical balloons that include a balloon wall that includes a composite material that includes a polymeric material and a filler within the polymeric material. The polymeric material includes a reaction product of a first polymeric material that includes first polymer chains having a plurality of spaced apart carboxylic acid groups, and a second polymeric material that includes second polymer chains having a plurality of spaced apart primary amino groups, hydroxyl groups, thiol groups, or mixtures of these groups. The amino groups, hydroxyl groups and thiol groups react with the carboxylic acid groups to form, respectively, amide, ester and thioester groups.

In another aspect, the disclosure features a medical balloon that includes a reaction product of chitosan and another polymer having carboxylic acid groups

Embodiments of the medical balloons can have one or more of the following features. The first polymeric material is or includes polyacrylic acid, polymethacrylic acid, poly(ethylene-co-acrylic acid), poly(2-ethylacrylic acid), poly(2-propylacrylic acid), melt blends of any of these or copolymers of any of these. The first polymeric material is or includes polyacrylic acid. The second polymeric material is or includes chitosan, poly[dimethylsiloxane-co-(3-aminopropyl)methylsiloxane], polyvinyl alcohol, poly(vinyl alcohol-co-vinyl acetate), poly(styrene-co-allyl alcohol), melt blends of any of these or copolymers of any of these. The second polymeric material is or includes chitosan. The first polymer chains each have between about 6 and about 10,000 carboxylic acid groups. A total number of primary amino groups, hydroxyl groups and/or thiol groups combined on each of the second polymer chains is between about 6 and about 10,000. A number average molecular weight of the first polymeric material is between about 2,500 and about 350,000, measured relative to polyethylene glycol standards in tetrahydrofuran, chloroform or chlorobenzene. A number average molecular weight of the second polymeric material is between about 2,500 and about 350,000, measured relative to polyethylene glycol standards in tetrahydrofuran, chloroform or chlorobenzene. The filler has a maximum dimension of less than 1000 nm. The filler is or includes allotropes of carbon, allotropes of carbon functionalized with hydrogen bonding groups (e.g., hydrogen bond acceptors and/or donors), metals, metal oxides, metalloid oxides, clays, ceramics or mixtures of any of these. The filler is or includes tubular structures made substantially of carbon. The tubular structures each have a single wall. The filler is or includes structures having hydrogen-bonding groups (e.g., acceptors and/or donors) extending from an outer portion of each structure. Each structure is more than 95 percent by weight carbon. The filler is dispersed substantially homogenously throughout the polymeric material. The filler is arranged in discrete longitudinal layers through a thickness of the composite material. The filler is or includes structures substantially spherical in shape, each having a diameter of less than 1000 nm. The filler is or includes a polymeric filler different from the first or second polymeric material, or the reaction product. The reaction product is or includes reacted first chains covalently bonded to reacted second chains by amide, ester or thioester bonds. The reacted first polymer chains are intermingled with reacted second polymer chains throughout a thickness of the composite material. The composite material has a tensile strength of greater than 40 MPa. The composite material has an electrical conductivity of greater than 50 S/cm. The composite material has voids therein. The composite material has a porosity of greater than 75 percent. The composite material is coated with a material that includes a therapeutic agent or includes a therapeutic agent therein. The composite material further includes a drug conjugate that can release a drug. The balloon is coated with a material that includes a therapeutic agent therein and/or thereon.

In another aspect, the disclosure features methods of making medical balloons that include providing a substrate and depositing first and second polymeric materials and/or a reaction product of the first and second polymeric materials onto the substrate. The first polymeric material includes first polymer chains having a plurality of spaced apart carboxylic acid groups, and the second polymeric material includes second polymer chains having a plurality of spaced apart primary amino groups, hydroxyl groups, thiol groups, or mixtures of these groups.

Embodiments of the methods can have one or more of the following features. The first and second polymeric materials are deposited concurrently. The first or second polymeric materials are deposited before the other polymeric material. The first and second polymeric materials are deposited by spraying the respective polymers onto the substrate from a solution or a dispersion of each respective polymer. The solution or dispersion of the first and/or second polymeric materials includes a filler having a maximum dimension of less than 1000 nm. The methods further include removing the substrate. The removing of the substrate includes melting the substrate. The substrate is or includes ice. The removing of the substrate includes dissolving the substrate.

Embodiments and/or aspects may include one or more of the following advantages. Balloons can be provided that are formed of a polymeric material that includes additives and particulates, e.g., carbon particulates, that are evenly dispersed, and not excessively aggregated, so that balloon properties are uniform and reproducible. Techniques for forming the balloons include combining two polymers, a dispersive polymer and a balloon polymer, by applying the polymers to a pre-form, the polymers being applied, e.g., concurrently from two separate solutions, or in a series of steps, e.g., two, three or five steps. Balloons can be provided in which properties, such as puncture resistance, scratch resistance, burst strength, tensile strength, porosity, drug release, and electrical and thermal conductivity, are enhanced for a given application. Balloons can be provided that are formed entirely of, or that include as part of their structure, composite materials that are highly loaded with a filler, e.g., carbon nanotubes. In such composites, the filler can be homogeneously dispersed throughout the composite, providing for uniformity in the properties of balloons formed of or carrying such composites. Balloons can be provided that are porous. Such porosity can be used to make drug-eluting balloons. Balloons can be provided that have a high electrical and/or thermal conductivity. For example, balloons can be provided that are formed entirely of, or that include as part of their structure, composite materials that have an electrical conductivity of greater than 50 S/cm, e.g., greater than 60, 70, 80, 90, 100, 175, 200, 250 or even greater than 300 S/cm. Also, for example, balloons can be provided that are formed entirely of, or that include as part of their structure, composite materials that have a high tensile strength, e.g., greater than 100 MPa, e.g., greater than 150, 250, or even greater than 300 MPa, enabling thin and ultra-thin walled balloons.

The details of one or more embodiments of the disclosure are set forth in the accompanying drawings, and in the description below. Other features and advantages will be apparent from the description and drawings, and from the claims.

### DESCRIPTION OF DRAWINGS

FIGS. 1A-1C are partial longitudinal cross-sectional views, illustrating delivery of a stent in a collapsed state (FIG 1A), expansion of the stent (FIG 1B), and deployment of the stent in a body lumen (FIG 1C).
FIG 2 is a highly enlarged, schematic view of the indicated region of FIG. 1B, illustrating a composite material that includes a polymeric material and a filler.
FIG 3 schematically illustrates the formation of the polymeric material of FIG 2.
FIGS. 4A-4E shows structures of specific examples of the first polymeric material.
FIGS. 5A and 5B, 6A and 6B, 7 and 8A-8D show structures of specific examples of the second polymeric material.
FIGS. 9A and 9B show schematic structures of tubular structures, e.g., carbon nanotubes, and functionalized tubular structures, e.g., carbon nanotubes functionalized with carboxylic acid groups.
FIG 10 is a series of schematic cross-sectional views, which collectively illustrate a method of forming a balloon that includes a wall formed of a composite in which reacted polyacrylic acid chains are intermingled with reacted chitosan chains throughout a thickness of the composite material.

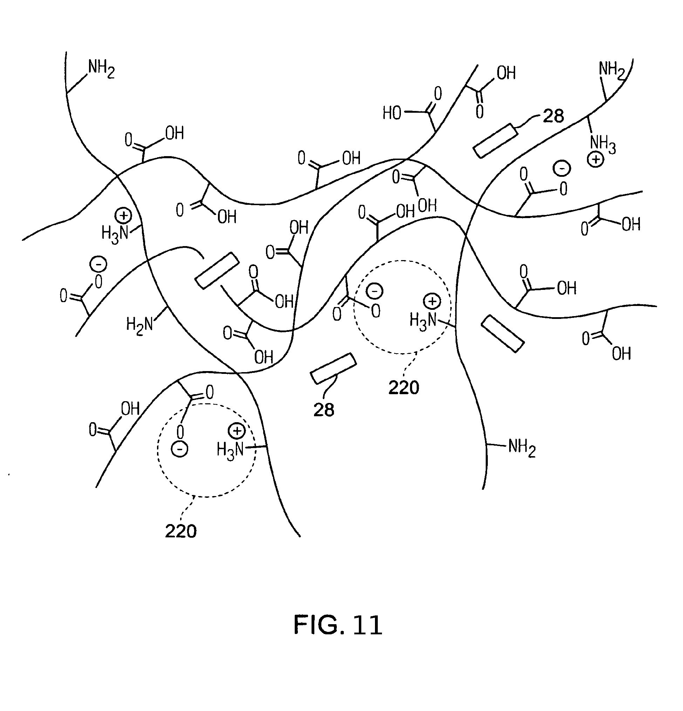

FIGS. 11 and 12 are schematic views of the indicated region of FIG 13, illustrating an acid/base reaction between intermingled chains of polyacrylic acid and chitosan that has a filler therein (FIG 11), and the same composite after heat treatment, illustrating amide linkages between reacted polyacrylic acid and chitosan portions (FIG. 12).

### DETAILED DESCRIPTION

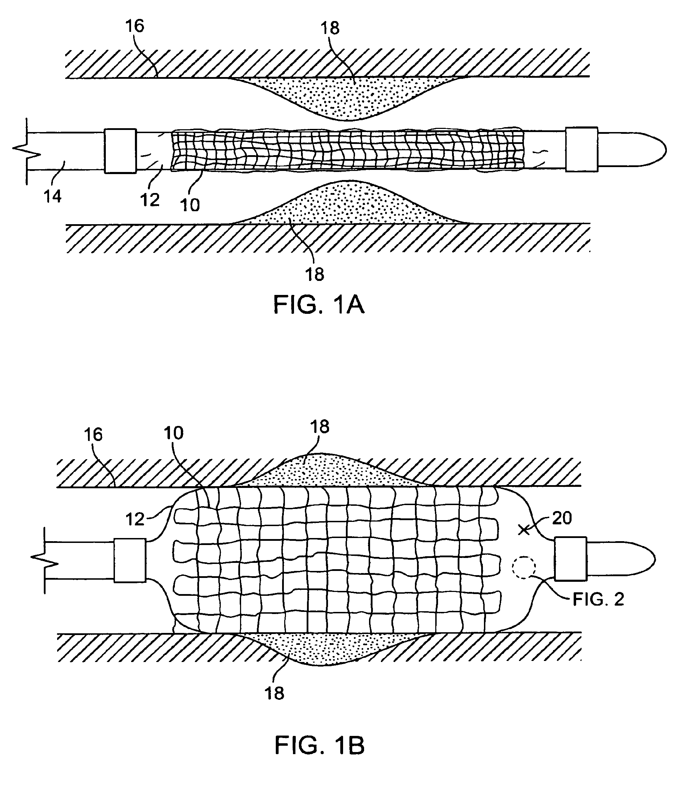

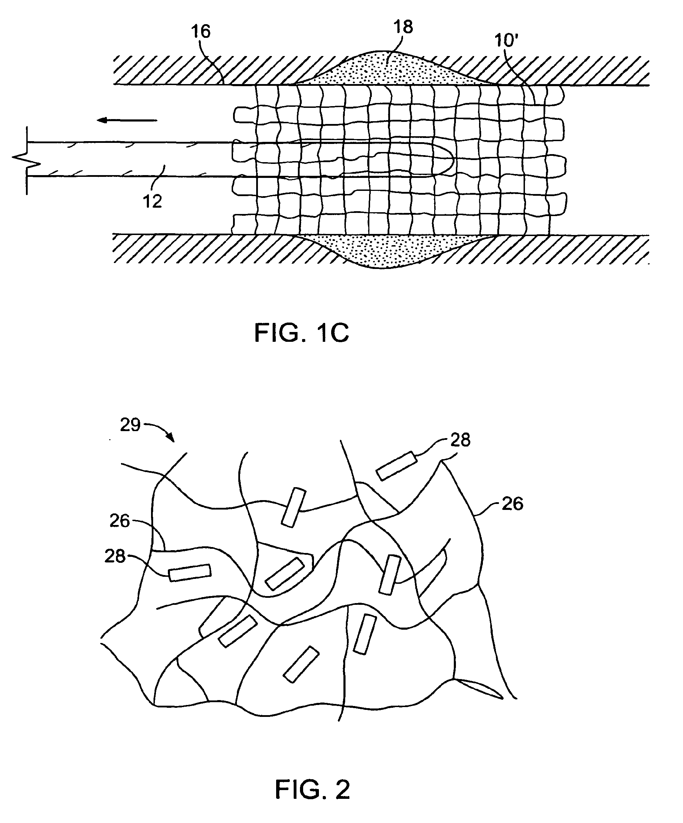

Referring to FIGS. 1A-1C, an unexpanded stent 10 is placed over a balloon 12 carried near a distal end of a catheter 14, and is directed through a lumen 16, e.g., a blood vessel such as the coronary artery, until the portion carrying the balloon and stent reaches the region of an occlusion 18 (FIG 1A). The stent is then radially expanded by inflating the balloon 12, and pressed against the vessel wall with the result that occlusion 18 is compressed, and the vessel wall surrounding it undergoes a radial expansion (FIG 1B). The pressure is then released from the balloon and the catheter is withdrawn from the vessel, leaving behind expanded stent 10' in the lumen (FIG 1C).

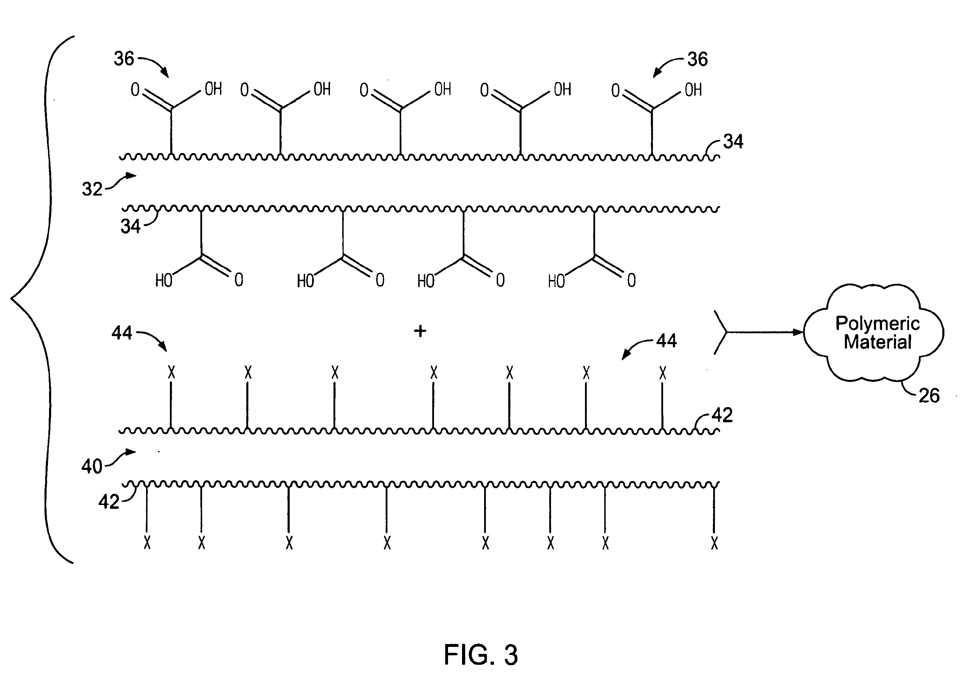

Referring also now to FIGS. 2 and 3, the balloon 12 includes a balloon wall 20 formed of a composite material 29 that includes a polymeric material 26 and a filler 28 uniformly dispersed within the polymeric material. Referring particularly to FIG. 3, the filler and/or additives, particularly carbon additives, such as nanotubes, are uniformly dispersed. The balloon can be formed using a polymer with good dispersion properties in combination with a balloon polymer that has properties particularly advantageous to balloons. The dispersive polymer can be, e.g., a nucleophilic polymer, such as a polymer having amino groups (e.g., primary amino groups, secondary amino groups, or tertiary amino groups), hydroxyl groups and/or thiol groups. Examples include biologically-derived polymers such as chitosan and DNA. The balloon polymer can be, e.g., an electrophilic polymer, such as one that includes electrophilic groups, e.g., carboxylic acid groups, that react, e.g., ionically or covalently, with the dispersive polymer. Examples include polyacrylic acid and polyethylene terephthalates, such as a carboxylic acid functionalized polyethylene terephthalate. The polymers can be applied and combined on a pre-fonn substrate, as will be discussed below. The polymeric material 26 includes a reaction product of a first balloon polymeric material 32 that includes first polymer chains 34 having a plurality of spaced apart carboxylic acid groups 36, and a second dispersive polymeric material 40 that includes second polymer chains 42 having a plurality of spaced apart groups 44 (X) that can react with carboxylic acid groups, such as amino groups (e.g., primary amino groups), hydroxyl groups, thiol groups, or mixtures of these groups.

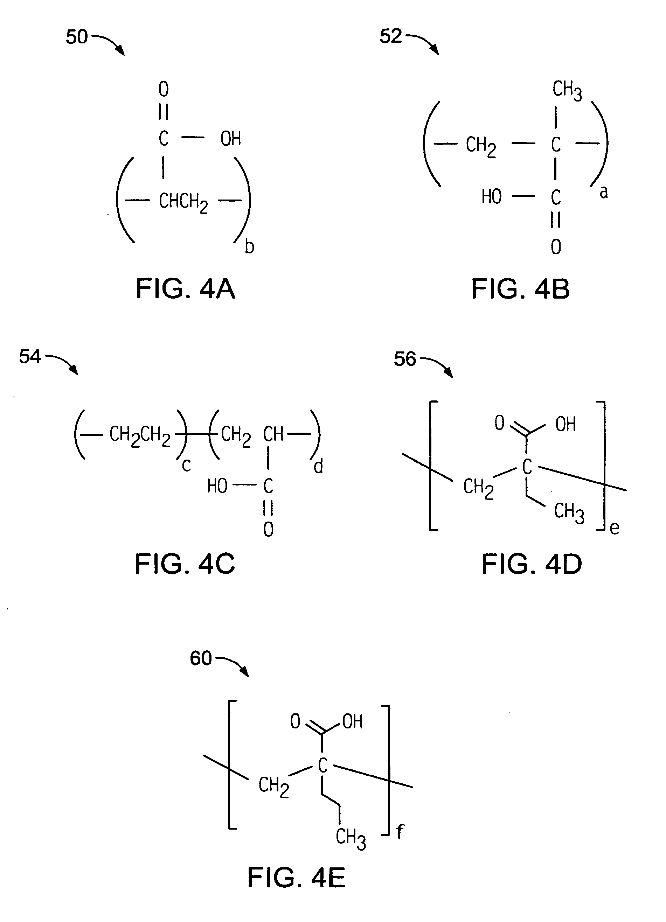

The balloon polymeric material is a material that has suitable balloon properties, e.g., mechanical properties such as tensile strength and reacts with the dispersive polymer. For example (and by reference to FIGS. 4A-4E), the first polymeric material can be or can include polyacrylic acid homopolymer (50, FIG. 4A), polymethacrylic acid homopolymer (52, FIG. 4B), poly(ethylene-co-acrylic acid) (54, FIG. 4C), poly(2-ethylacrylic acid) homopolymer (56, FIG. 4D), poly(2-propylacrylic acid) homopolymer (60, FIG. 4E), or blends of any of these polymers. In embodiments, a, b, c, d, e and f are between about 5 and about 6,500, e.g., between about 15 and about 3,500, or between about 50 and about 3,000. In embodiments, the ratio of c to d is between about 1/10 to about 7/10, e.g., between about 2/10 and about 5/10. In embodiments, a, b, c, d, e, and f are chosen such that a number average molecular weight of the first polymeric material is between about 2,500 and about 350,000, measured relative to mono-disperse polyethylene glycol standards in tetrahydrofuran, chloroform or chlorobenzene. In embodiments, the first polymer chains each have between about 6 and about 10,000 carboxylic acid groups, e.g., between about 10 and about 7,500, or between about 25 and about 5,000 carboxylic acid groups.

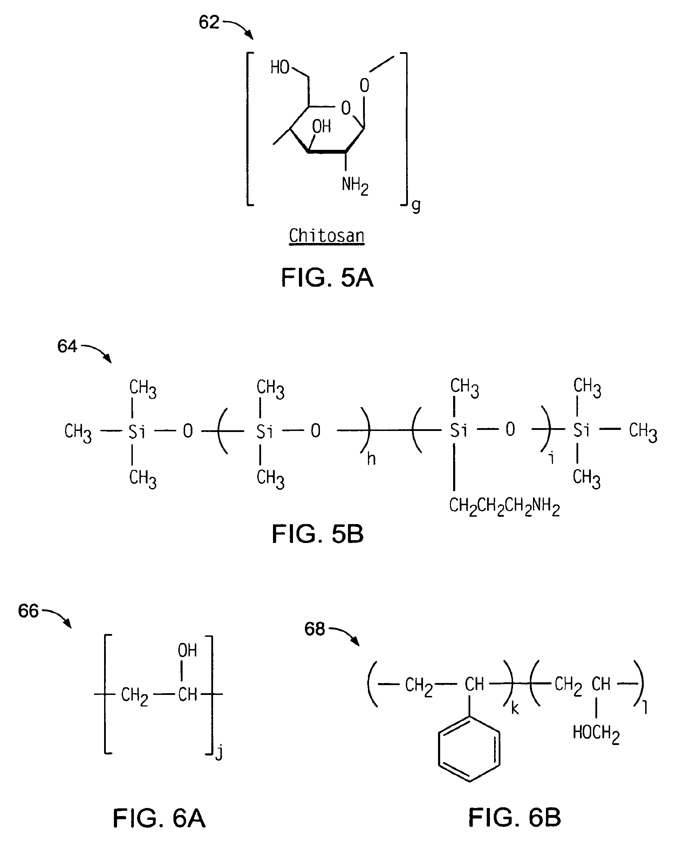

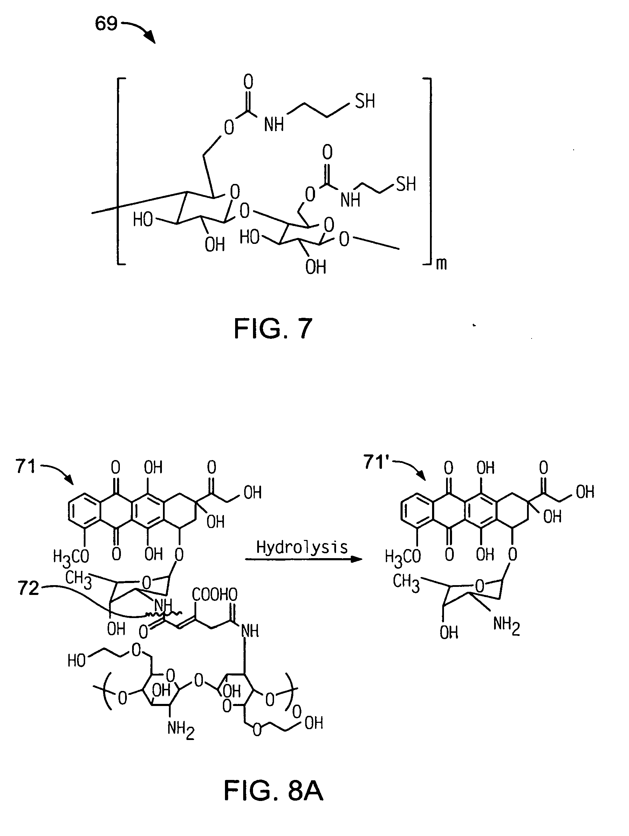

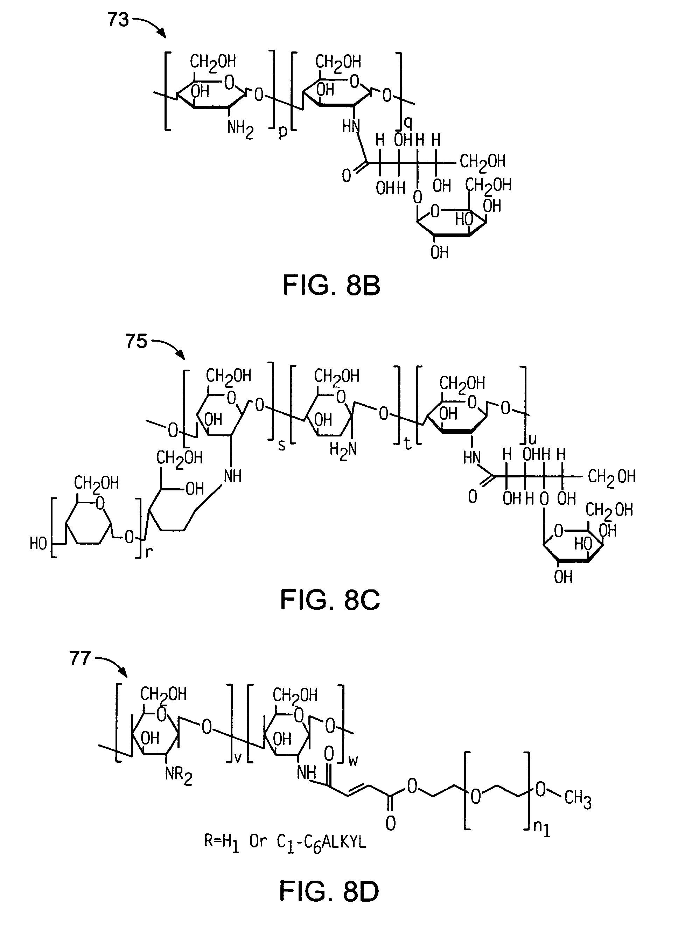

The dispersive polymer enhances dispersion of additives and reduces aggregation. For example (and by reference to FIGS. 5A and 5B, 6A and 6B, 7 and 8A-8D), the second polymeric material can be or can include chitosan homopolymer (62, FIG. 5A), poly[dimethylsiloxane-co-(3-aminopropyl)methylsiloxane] (64, FIG. 5B), polyvinyl alcohol (66, FIG. 6A), e.g., 99 percent hydrolyzed polyvinyl alcohol, poly(vinyl alcohol-co-vinyl acetate), poly(styrene-co-allyl alcohol) (68, FIG. 6B), e.g., having 40 mole percent allyl alcohol, carbohydrates and carbohydrate derivatives (69, FIG. 7), such as those described by Stamler et al. in U.S. Patent No. 6,875,840, doxorubicin conjugated glycol-chitosan polymers (71, FIG. 8A), such as those described by Son et al., Journal of Controlled Release, 91, 135-145 (2003), galactosylated chitosans (73, FIG. 8B) and galactosylated chitosans-graft-dextrans (75, FIG. 8C), such as those described by Park et al., Journal of Controlled Release, 69, 97-108 (2000), poly(ethylene glycol)-graft-trimethyl chitosan copolymers (77, FIG. 80), such as those described by Mao et al., Biomaterials, 26, 6343-6356 (2005), or blends of any of these polymers. In embodiments, g, h, i, j, k, l, m, o, p, q, r, s, t, u, v, w and n₁ are between about 5 and about 6,500, e.g., between about 15 and about 3,500, or between about 50 and about 3,000. In embodiments, a ratio of h to i or k to 1 is between about 1/10 to about 7/10, e.g., between about 1/10 and about 5/10. In embodiments, a ratio ofp to q, t to (r + s + u), or v to w is, e.g., from about 10/1 to about 10/5, e.g., between about 10/1 to about 10/4. In embodiments, g, h, i, j, k, l, m, o, p, q, r, s, t, u, v, w and n₁ are chosen such that a number average molecular weight of the second polymeric material is between about 2,500 and about 350,000, measured relative to mono-disperse polyethylene glycol standards in tetrahydrofuran, chloroform or chlorobenzene. In embodiments, a total number of primary amino groups, hydroxyl groups and/or thiol groups combined on each of the second polymer chains is between about 6 and about 10,000, e.g., between about 10 and about 7,500, or between about 25 and about 5,000. In some instances, having the second polymeric material be or include a therapeutic agent conjugate can be advantageous for delivering a therapeutic agent during deployment of a medical device, such as a stent, and/or during expansion of the balloon within a lumen. Referring particularly now to FIG. 8A, doxorubicin conjugated glycol-chitosan polymers (71) can hydrolyze *in-vivo* at amide linkage 72, releasing doxorubicin (71'). Other suitable dispersive polymers include DNA. Dispersability can be quantified and/or monitored, e.g., by using light scattering or by rheological means, e.g., as described by Huang, Physical Review B 73, 125422 (2006).

The filler can be, e.g., an allotrope of carbon (e.g., diamond, graphite, C60, C70, C540, a single or multi-wall carbon tube, or amorphous carbon), a functionalized allotrope of carbon (e.g., functionalized with hydrogen bonding groups such as hydrogen bond acceptors and/or donors), a metal, a metal oxide (e.g., titanium dioxide), a metalloid oxide (e.g., silicon dioxide), a clay (e.g., kaolin), a ceramic (e.g., silicon carbide or titanium nitride), a polymeric material, different from the first or second polymeric material or a reaction product of the first and second polymeric materials, or mixtures or any of these fillers. If desired, the carbon nanotubes can encapsulate atoms other than carbon, such as a metal, which can, e.g., enhance radiopacity.

In embodiments, the composite includes, e.g., between about 10 percent by weight filler and about 70 percent by weight filler, e.g., between about 20 percent and about 60 percent, or between about 30 percent and about 58 percent by weight filler. When flexibility is a desirable attribute, it is often desirable to maintain the amount of filler to less than about 20 weight percent.

In embodiments, some of the filler particles have a maximum dimension of not more than about 1000 nm, e.g., not more than about 750 nm, 600 nm, 500 nm, 400 nm, 250 nm, 150 nm, or not more than about 100 nm. In embodiments, each filler particle has a maximum dimension of between about 100 nm and about 1000 nm, e.g., between about 150 nm and about 800 nm, or between about 200 nm and 600 nm.

In embodiments, the filler includes structures having hydrogen-bonding groups extending from an outer portion of each structure, e.g., carboxylic acid groups, amide groups, hydroxyl groups, or silinol groups. These groups can aid in the dispersion of the particles in solution during preparation of the composite.

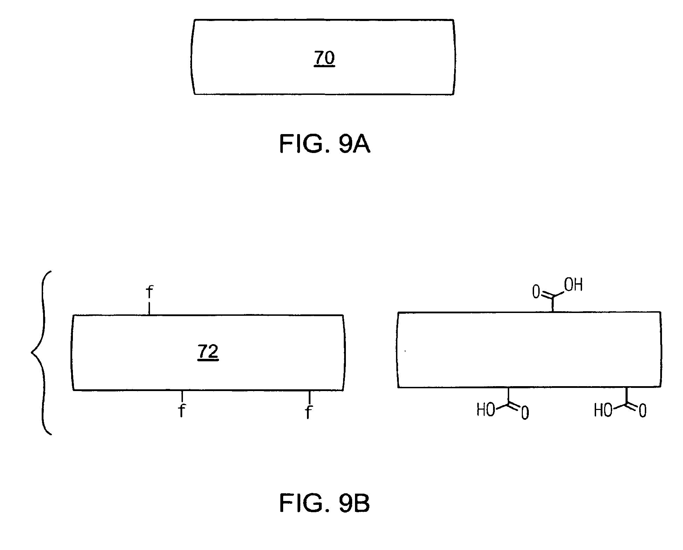

Referring now to FIGS. 9A-9B, in embodiments, some or all of the filler particles (70, FIG. 9) are tubular in shape, e.g., containing greater than 90 percent by weight carbon, e.g., greater than 91, 93, 95, 97, or even greater than 99 percent carbon by weight. In embodiments, each filler particle is tubular in shape, and is formed substantially of carbon, having only bound hydrogen at boundaries of the each tubular structure. In embodiments, the filler includes tubular structures (72, FIG. 9B), such as carbon nanotubes, having an outer portion that has been functionalized with a group (*f*), such as a hydrogen-bonding group like a carboxylic acid group.

In embodiments, the filler includes carbon nanotubes that are functionalized and that have an overall diameter, e.g., between about 3 nm and about 25 nm, e.g., between about 4 nm and about 10 nm. In embodiments, the carbon nanotubes can have a length of between about 300 nm and about 1600 nm, e.g., between about 500 nm and about 1000 nm.

Carbon nanotubes, and some of their properties, including dipersibility in a solvent are described in more detail by Moulton et al., Carbon, 43, 1879-1884 (2005); Jiang et al., Electrochemistry Communications, 7, 597-601 (2005); and Shim et al., Langmuir, 21(21), 9381-9385 (2005).

If desired, the filler can include structures that are substantially spherical in shape, each having a diameter of less than about 1000 nm, e.g., less than about 750 nm, 600 nm, 500 nm, 350 nm, 200 nm, 125 nm or even less than 75 nm. In embodiments, the filler can include structures that each have a diameter between about 50 nm and about 900 nm, e.g., between about 100 nm and about 750 nm or between about 250 nm and about 600 nm. In embodiments, blends of fillers, such as blends of tubular structures and spherical structures are utilized.

In embodiments, the reaction product features an ionic complex between the first and second polymeric materials, such as an acid/base adduct of the first and second polymeric materials. Ionic complexes are described by Wang et al., Journal Applied Polymer Science, 65, 1445-1450 (1997); and Palloma et al., Biomalerials, 24, 1459-1468 (2003). In other embodiments, the reaction product features reacted first chains, which are covalently bonded to reacted second chains by amide, ester and/or thioester bonds. In still other embodiments, the reaction product includes portions that are ionic in nature, and portions which include reacted first chains covalently bonded to reacted second chains. Reaction products that include both ionic complex portions and covalently bonded portions are described by Berger et al., European Journal of Pharmaceutics and Biopharmaceutics, 57, 19-34 (2004).

The reacted first and second polymeric chains of the composite can take on a variety of forms and structures. For example, the reacted first polymer chains can be intermingled with reacted second polymer chains throughout a thickness of the composite material, or the reacted first and second polymeric materials can form distinct layers, e.g., alternating layers throughout a thickness of the composite material. In a specific example, the composite material includes a first layer that includes reacted first polymeric material, and a second layer that includes reacted second polymeric material having filler dispersed therein. If desired, e.g., for enhanced mechanical properties, the first layer can also have a filler dispersed therein. Also, if desired, the composite material can further include a third layer that includes a second polymeric material that, optionally, has a filler dispersed therein.

The composite material can have a high tensile strength, e.g., the tensile strength can be greater than about 40 MPa, e.g., greater than about 50 MPa, 75 MPa, 100 MPa, or even greater than about 150 MPa. In addition, the composite material can have a high electrical conductivity, e.g., greater than about 50 S/cm, e.g., greater than about 60 S/cm, 75 S/cm, 100 S/cm, 150 S/cm, 200 S/cm, even greater than about 300 S/cm.

In embodiments, the composite material has a tensile strength of greater than about 60 MPa and an electrical conductivity of greater than about 100 S/cm, or greater than about 100MPa and a conductivity of greater than about 300 S/cm.

If desired, the composite can have voids, e.g., interconnected voids, that provide a porous composite. For example, the voids can have a maximum dimension that is greater than 500 nm, e.g., greater than 750 nm, 1,000 nm, 1,500 nm, or even greater than 2,500 nm. The voids can provide a porosity that is, e.g., greater than 75 percent, e.g., greater than 80 percent, 85 percent, 90 percent, or even greater than 95 percent, as measured using mercury porosimetry. If desired, the porous structure can be filled with a therapeutic agent so that the agent can be delivered from the balloon during deployment of a medical device.

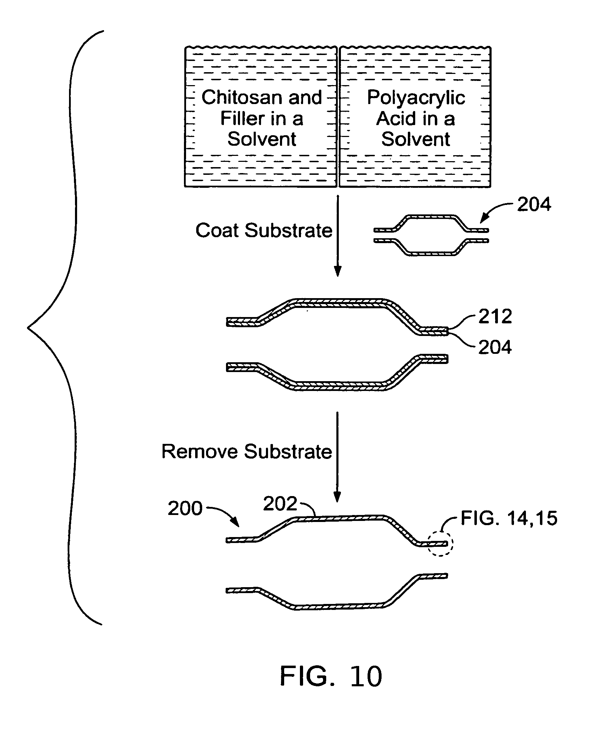

Referring now to FIG. 10 a balloon 200 that includes a wall 202 formed of a composite material having an intermingled structure of reacted chitosan (62)/filler and polyacrylic acid (50) can be made by concurrently depositing chitosan/filler and polyacrylic acid as two separate streams of material. This can be achieved by forming a chitosan filler dispersion by dissolving chitosan in a solvent, at a desired concentration, and then adding to the chitosan in solution a filler at a desired loading to form a homogeneous dispersion of the chitosan and the filler; forming a polyacrylic acid solution in a solvent at a desired concentration; and then concurrently applying the chitosan/filler and polyacrylic acid to a substrate 204. Once the chitosan/filler and polyacrylic acid has been deposited, the solvent is removed from the deposited materials, forming a layer 212 of the chitosan/filler/polyacrylic acid about the substrate 204. After the solvent is removed from the deposited coating, the substrate is removed, e.g., by dissolving the substrate in a solvent. After removal of the substrate, a chitosan/filler/polyacrylic acid composite is provided. Heat treatment provides completed balloon 200 having a wall 202 that includes a composite formed of a reaction product of chitosan/filler and polyacrylic acid.

In some embodiments, a porous balloon is provided by including a water-soluble filler, e.g., sodium chloride or glucose, in the chitosan solution in an anhydrous solvent and/or the polyacrylic acid solution in an anhydrous solvent. After applying the solutions, removing the solvent and heat treating, the water-soluble material can be removed from the formed balloon, e.g., by sonicating in a water bath to provide a porous structure.

In other embodiments, during the formation of the balloon, one or more cutting members are attached to the balloon to form a cutting balloon. Cutting balloons are described in O'Brien U.S. Patent No. 7,070,576 and Radisch, U.S. Patent No. 7,011,670.

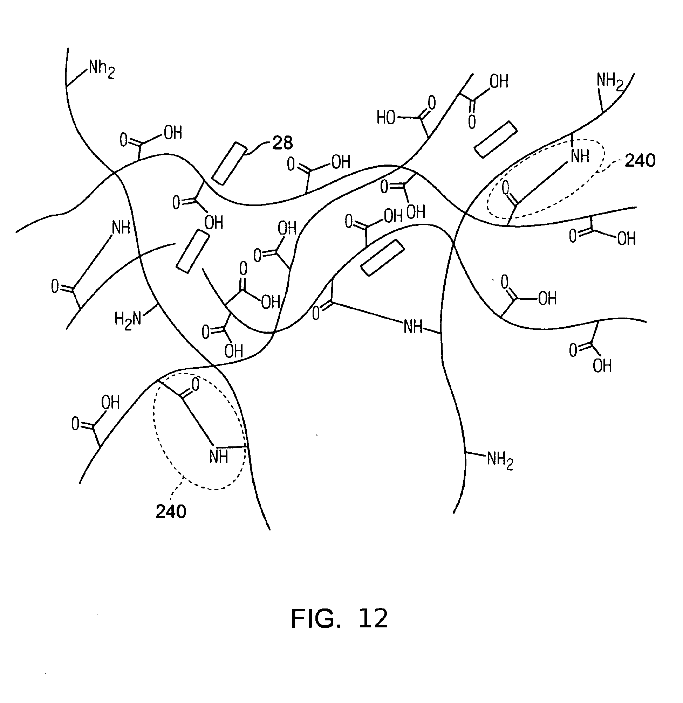

Referring now as well to FIG, 11 for a more detailed view of a portion of the balloon wall structure made by the method discussed in reference to FIG. 10. Prior to heat treatment, reacted polyacrylic acid portions include carboxylate groups and chitosan portions include ammonium groups. Ion pairs 220 aid in holding the composite together. Referring now as well to FIG. 12. after heat treatment, amide linkages 240 form between reacted chains of chitosan and reacted chains of polyacrylic acid.

Any of the composites described herein can be made porous by incorporating dissolvable polymeric particles, e.g., having a diameter of from about 500 nm to about 2,500 nm, into the composites during their formation. Removal of the particles can be achieved by dissolving the particles with a solvent from the formed composites.

Any component of any balloon can include a therapeutic agent therein and/or thereon. Also, any balloon can be coated with a material that includes a therapeutic agent therein and/or thereon. Electroporation and iontophoresis can be used to assist in the delivery of a therapeutic agent. For example, when a therapeutic agent is utilized in a conductive composite, delivery of the therapeutic can be aided by applying an electric field to the conductive composite, e.g., between about 5 V/cm and about 2.5 kV/cm, between about 25 V/cm and about 1.5 kV/cm or between about 50 kV/cm and about 1 kV/cm. In some embodiments, the electric field is applied in a pulsing manner. For example, the pulse length can be from about 50 *µs* to about 30 ms, from about 100 *µs* to about 25 ms or from about 150 *µs* to about 20 ms. Generally, electroporation is described by Davalos et al., Microscale Thermophysical Engineering, 4:147-159 (2000*).* A power supply for a pulsed power supply for electroporation has been described by Grenier, a thesis presented to the University of Waterloo, Ontario, Canada, in a work entitled "Design of a MOSFET-Based Pulsed Power Supply for Electroporation" (2006).

In general, the therapeutic agent can be a genetic therapeutic agent, a non-genetic therapeutic agent, or cells. Therapeutic agents can be used singularly, or in combination. Therapeutic agents can be, e.g., nonionic, or they may be anionic and/or cationic in nature. One therapeutic agent for a vascular application is one that inhibits restenosis. A specific example of one such therapeutic agent that inhibits restenosis is paclitaxel or derivatives thereof, e.g., docetaxel. Soluble paclitaxel derivatives can be made by tethering solubilizing moieties off the 2' hydroxyl group of paclitaxel, such as -COCH₂CH₂CONHCH₂CH₂(OCH₂)ₙOCH₃ (n being, e.g., 1 to about 100 or more). Li et al., U.S. Patent No. 6,730,699 describes additional water soluble derivatives of paclitaxel.

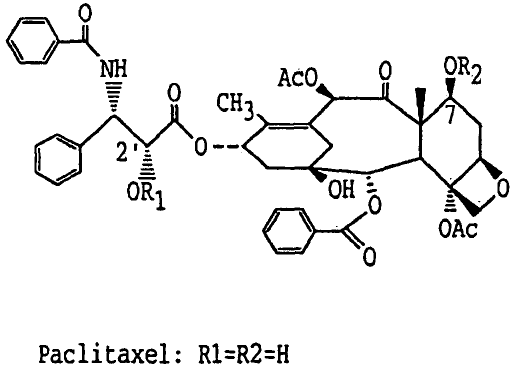

Exemplary non-genetic therapeutic agents include: (a) anti-thrombotic agents such as heparin, heparin derivatives, urokinase, PPack (dextrophenylalanine proline arginine chloromethylketone), and tyrosine; (b) anti-inflammatory agents, including non-steroidal anti-inflammatory agents (NSAID), such as dexamethasone, prednisolone, corticosterone, budesonide, estrogen, sulfasalazine and mesalamine; (c) anti-neoplasticlantiproliferative/anti-miotic agents such as paclitaxel, 5-fluorouracil, cisplatin, vinblastine, vincristine, epothilones, endostatin, angiostatin, angiopeptin, rapamycin (sirolimus), biolimus, tacrolimus, everolimus, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, antiplatelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines, (r) hormones; and (s) antispasmodic agents, such as alibendol, ambucetamide, aminopromazine, apoatropine, bevonium methyl sulfate, bietamiverine, butaverine, butropium bromide, n-butylscopolammonium bromide, caroverine, cimetropium bromide, cinnamedrine, clebopride, coniine hydrobromide, coniine hydrochloride, cyclonium iodide, difemerine, diisopromine, dioxaphetyl butyrate, diponium bromide, drofenine, emepronium bromide, ethaverine, feclemine, fenalamide, fenoverine, fenpiprane, fenpiverinium bromide, fentonium bromide, flavoxate, flopropione, gluconic acid, guaiactamine, hydramitrazine, hymecromone, leiopyrrole, mebeverine, moxaverine, nafiverine, octamylamine, octaverine, oxybutynin chloride, pentapiperide, phenamacide hydrochloride, phloroglucinol, pinaverium bromide, piperilate, pipoxolan hydrochloride, pramiverin, prifinium bromide, properidine, propivane, propyromazine, prozapine, racefemine, rociverine, spasmolytol, stilonium iodide, sultroponium, tiemonium iodide, tiquizium bromide, tiropramide, trepibutone, tricromyl, trifolium, trimebutine, tropenzile, trospium chloride, xenytropium bromide, ketorolac, and pharmaceutically acceptable salts thereof.

Exemplary genetic therapeutic agents include anti-sense DNA and RNA as well as DNA coding for: (a) anti-sense RNA, (b) tRNA or rRNA to replace defective or deficient endogenous molecules, (c) angiogenic factors including growth factors such as acidic and basic fibroblast growth factors, vascular endothelial growth factor, epidermal growth factor, transforming growth factor *α* and *β*, platelet-derived endothelial growth factor, platelet-derived growth factor, tumor necrosis factor *α*, hepatocyte growth factor and insulin-like growth factor, (d) cell cycle inhibitors including CD inhibitors, and (c) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers PVP, SP1017 (SUPRATEK), lipids such as cationic lipids, liposomes, lipoplexes, nanoparticles, or micro particles, with and without targeting sequences such as the protein transduction domain (PTD).

### OTHER EMBODIMENTS

A number of embodiments have been described.

While FIG. 10 illustrates balloons in which the entire balloon wall is formed of the composite, in some embodiments, only a portion of the balloon wall is made of the composite. For example, the composite can be about another material, e.g., a polyetheramide, such a those available under the tradename PEBAX^{®}.

The balloon formed can be of any desired longitudinal or transverse cross-section by selecting a corresponding substrate. For example, the substrate can be selected to provide a balloon suitable for delivering a bifurcated endoprosthesis. For example, a substrate can be used having one or more "bulges".

Still other embodiments are in the following claims.

## Claims

1. A medical balloon comprising: a balloon wall comprising a composite material comprising
a polymeric material; and
a filler within the polymeric material,
wherein the polymeric material comprises a reaction product of a first polymeric material comprising first polymer chains having a plurality of carboxylic acid groups, a second polymeric material comprising second polymer chains having a plurality of amino groups, hydroxyl groups, thiol groups, or mixtures of these groups, the reaction product comprising reacted first chains covalently bonded to reacted second chains by amide, ester and/or thioester bonds, wherein the first polymeric material is selected from the group consisting of polyacrylic acid, polymethacrylic acid, poly(ethylene-co-acrylic acid), poly(2-ethylacrylic acid), poly(2-propylacrylic acid) and mixtures thereof, and wherein the second polymeric material is selected from the group consisting of chitosan, poly[dimethylsiloxane-co-(3- aminopropyl)methylsiloxane], polyvinyl alcohol, poly( vinyl alcohol-co-vinyl acetate), poly(styrene-co-allyl alcohol) and mixtures thereof.

2. The medical balloon of claim 1, wherein the first polymer chains each have between about 6 and about 10,000 carboxylic acid groups.

3. The medical balloon of claim 1, wherein a total number of primary amino groups, hydroxyl groups and/or thiol groups combined on each of the second polymer chains is between about 6 and about 10,000.

4. The medical balloon of claim 1, wherein a number average molecular weight of the first polymeric material is between about 2,500 and about 350,000, measured relative to polyethylene glycol standards.

5. The medical balloon of claim 1, wherein a number average molecular weight of the second polymeric material is between about 2,500 and about 350,000, measured relative to polyethylene glycol standards.

6. The medical balloon of claim 1, wherein the filler is selected from the group consisting of allotropes of carbon, allotropes of carbon functionalized with hydrogen bonding groups, metals, metal oxides, metalloid oxides, clays, ceramics and mixtures thereof.

7. The medical balloon of claim 1, wherein the filler is dispersed substantially homogenously throughout the polymeric material.

8. The medical balloon of claim 1, wherein the filler is arranged in discrete longitudinal layers through a thickness of the composite material.

9. The medical balloon of claim 1, wherein reacted first polymer chains are intermingled with reacted second polymer chains throughout a thickness of the composite material.

## Patentansprüche

1. Ein medizinischer Ballon umfassend: eine Ballonwand, die Verbundmaterial umfasst, welches ein Polymermaterial und
einen Füllstoff innerhalb des Polymermaterials umfasst,
wobei das Polymermaterial ein Reaktionsprodukt eines ersten Polymermaterials umfasst, das erste Polymerketten, die eine Vielzahl an Karboxylsäuregruppen aufweisen, umfasst,
ein zweites Polymermaterial, das zweite Polymerketten umfasst, die eine Vielzahl an Aminogruppen, Hydroxylgruppen, Thiolgruppen oder Mischungen aus diesen Gruppen aufweist,
das Reaktionsprodukt abreagierte erste Ketten umfasst, die über Amid-, Ester- und/oder Thioester- Bindungen kovalent an abreagierte zweite Ketten gebunden sind, wobei das erste Polymermaterial aus der Gruppe bestehend aus Polyacrylsäure, Polymethacrylsäure, Poly(Ethylen-co-Acrylsäure), Poly(2-Ethylacrylsäure), Poly(2- Propylacrylsäure) und Mischungen davon ausgewählt ist und wobei
das zweite Polymermaterial aus der Gruppe bestehend aus Chitosan, Poly[Dimethylsiloxan-co-(3-Aminopropyl)methylsiloxan], Polyvinylalkohol, Poly(vinylalkohol-co-Vinylacetat), Poly(styrol-co-allylalkohol) und Mischungen davon ausgewählt ist.

2. Der medizinische Ballon nach Anspruch 1, wobei die ersten Polymerketten jeweils zwischen etwa 6 und etwa 10000 Karboxylsäuregruppen aufweisen.

3. Der medizinische Ballon nach Anspruch 1, wobei eine Gesamtzahl der primären Aminogruppen, Hydroxylgruppen und/oder Thiolgruppen kombiniert mit jeder der zweiten Polymerketten zwischen etwa 6 und etwa 10000 liegt.

4. Der medizinische Ballon nach Anspruch 1, wobei eine Zahl durchschnittlicher Molmasse des ersten Polymermaterials zwischen etwa 2500 und etwa 350000 relativ gemessen zum Polyethylenglykol Standard liegt.

5. Der medizinische Ballon nach Anspruch 1, wobei eine Zahl durchschnittlicher Molmasse des zweiten Polymermaterials zwischen etwa 2500 und etwa 350000 relativ gemessen zum Polyethylenglykol Standard liegt.

6. Der medizinische Ballon nach Anspruch 1, wobei der Füllstoff aus der Gruppe bestehend aus Kohlenstoff-Allotropen, Kohlenstoff-Allotropen funktionalisiert mit Wasserstoffbindungsgruppen, Metallen, Metalloxiden, Halbmetalloxiden, Ton, Keramiken und Mischungen daraus ausgewählt ist.

7. Der medizinische Ballon nach Anspruch 1, wobei der Füllstoff im Wesentlichen homogen durch das Polymermaterial hindurch dispergiert ist.

8. Der medizinische Ballon nach Anspruch 1, wobei der Füllstoff in diskreten longitudinalen Schichten über eine Dicke des Verbundmaterials angeordnet ist.

9. Der medizinische Ballon nach Anspruch 1, wobei abreagierte erste Polymerketten mit abreagierten zweiten Polymerketten über eine Dicke des Verbundmaterials hindurch vermischt sind.

## Revendications

1. Ballonnet à usage médical, comprenant : une paroi de ballonnet comprenant un matériau composite comprenant
un matériau polymère ; et
une matière de remplissage à l'intérieur du matériau polymère,
le matériau polymère comprenant un produit de réaction d'un premier matériau polymère comprenant des premières chaînes polymères ayant une pluralité de groupes acide carboxylique, un deuxième matériau polymère comprenant des deuxièmes chaînes polymères ayant une pluralité de groupes amino, groupes hydroxyle, groupes thiol ou des mélanges de ces groupes, le produit de réaction comprenant des premières chaînes ayant réagi liées par covalence à des deuxièmes chaînes ayant réagi par des liaisons amide, ester et/ou thioester, le premier matériau polymère étant sélectionné parmi le groupe constitué d'acide polyacrylique, acide polyméthacrylique, poly(éthlylène-co-acide acrylique), poly(acide 2-éthylacrylique), poly(acide 2-propylacrylique) et des mélanges de ceux-ci, et le deuxième matériau polymère étant sélectionné parmi le groupe constitué de chitosane, poly[diméthylsiloxane-co-(3- aminopropyl)méthyl-siloxane), polyalcool vinylique, poly(alcool vinylique co-acétate vinylique), poly(styrène-co- alcool d'allyle) et des mélanges de ceux-ci.

2. Ballonnet à usage médical selon la revendication 1, dans lequel les premières chaînes polymères ont chacune entre environ 6 et environ 10 000 groupes acide carboxylique.

3. Ballonnet à usage médical selon la revendication 1, dans lequel un nombre total de groupes amino primaires, de groupes hydroxyle et/ou de groupes thiol combinés sur chacune des deuxièmes chaînes polymères est compris entre environ 6 et environ 10 000.

4. Ballonnet à usage médical selon la revendication 1, dans lequel un poids moléculaire moyen en nombre du premier matériau polymère est compris entre environ 2 500 et environ 350 000, mesuré par rapport à des standards polyéthylène glycol.

5. Ballonnet à usage médical selon la revendication 1, dans lequel un poids moléculaire moyen en nombre du deuxième matériau polymère est compris entre environ 2 500 et environ 350 000, mesuré par rapport à des standards polyéthylène glycol.

6. Ballonnet à usage médical selon la revendication 1, dans lequel la matière de remplissage est sélectionnée parmi le groupe constitué d'allotropes de carbone, d'allotropes de carbone fonctionnalisés avec des groupes de liaison hydrogène, des métaux, des oxydes de métaux, des oxydes de métalloïdes, des argiles, des céramiques et des mélanges de ceux-ci.

7. Ballonnet à usage médical selon la revendication 1, dans lequel la matière de remplissage est dispersée de façon sensiblement homogène à travers le matériau polymère.

8. Ballonnet à usage médical selon la revendication 1, dans lequel la matière de remplissage est agencée en couches longitudinales discrètes à travers une épaisseur du matériau composite.

9. Ballonnet à usage médical selon la revendication 1, dans lequel des premières chaînes polymères ayant réagi sont entrelacées avec des deuxièmes chaînes polymères ayant réagi à travers une épaisseur du matériau composite.
